# DEMANDE DE BREVET EUROPEEN

(11) **EP 3 878 431 A1**
(43) Date de publication de la demande: **15.09.2021**
(21) Numéro de dépôt: 21162176.8
(22) Date de dépôt: 11.03.2021
(51) Int. Cl.: A61K 8/06, A61K 8/25, A61K 8/27, A61K 8/34, A61K 8/365, A61K 8/37, A61K 8/60, A61Q 15/00

(54) **COMPOSITION COSMETIQUE DEODORANTE COMPRENANT DU ZINC RICINOLEATE**

(30) Priorité: 11.03.2020 FR 2002435
(71) Demandeur: Hyteck, 75006 Paris (FR)
(72) Inventeur: VAUSSELIN, Anne, 83700 SAINT-RAPHAEL (FR); RUBERTI, Pascale, 13122 VENTABREN (FR)
(74) Mandataire: Tripoz, Inès

(57) **Abrégé**

La présente invention concerne un composition cosmétique déodorante sous forme d'émulsion stable apte à être intégrée dans un déodorant sous forme de bille ou roll-on comprenant du zinc ricinoléate à une teneur supérieure à 1% caractérisée en ce qu'elle ne contient pas de sels d'aluminium, par exemple l'chlorhydrate d'aluminium.
La présente invention concerne également un déodorant comprenant une composition cosmétique déodorante selon l'invention.
La présente invention concerne également un procédé de stabilisation d'une émulsion apte à être intégrée dans un déodorant sous forme de bille ou roll-on comprenant une teneur en zinc ricinoléate supérieure à 1% caractérisé en ce qu'il comprend l'ajout d'au moins un émulsifiant huile dans eau à ladite émulsion.

## Description

La présente invention est relative au domaine des cosmétiques, plus particulièrement des déodorants.

La présente invention à trait plus particulièrement à une composition qui est une base déodorante neutre « BIO » 100 % constituée d'ingrédients biologiques et naturels.

Cette base est formulée prête à l'emploi et peut être utilisée pour fabriquer un déodorant ou être personnalisée par l'ajout d'ingrédients.

Les déodorants comprenant des substances entièrement d'origine naturelle appelés couramment déodorants « BIO », prennent une place de plus en plus importante sur le marché du fait de la demande des consommateurs qui souhaitent utiliser des produits ne contenant pas de composés issus de la pétrochimie ainsi que des composés sans dangers pour la santé. Des déodorants « BIO » prêts à l'emploi sont disponibles sur le marché.

Il existe également depuis quelques années une tendance à la préparation et à la fabrication de produits cosmétiques « maison ». Pour cela différentes recettes dites « maison » sont disponibles pour les consommateurs qui souhaitent fabriquer leur propres produits cosmétiques en suivant une recette simple à comprendre et facile à mettre en œuvre. Des recettes « maison » pour la fabrication de déodorants bio sont disponibles dans des revues spécialisées ainsi que sur internet, notamment les recettes mises au point par la société HYTECH AROMA-ZONE.

Différents actifs déodorants sont en général utilisés comme ingrédients principaux des déodorants, Le Zinc ricinoléate et le Triéthyl citrate sont par exemple utilisés dans les déodorants « BIO » car certifiés conformes aux normes régissant les compositions cosmétiques « BIO » par des organismes de certifications tel que ECOCERT.

Le Zinc ricinoléate est un ingrédient naturel obtenu à partir d'acide ricinoléique dérivé de l'huile de ricin et d'oxyde de zinc d'origine minérale. Il agit en tant que neutralisateur d'odeur en complexant les molécules malodorantes. Le Zinc ricinoléate est utilisé dans des compositions déodorantes que ce soit dans des applications textiles, traitement de l'air ou cosmétiques.

L'action du Zinc ricinoléate est divulgué dans le document ZA9607671 qui divulgue une composition sous forme de spray pour purifier l'air comprenant du zinc ricinoléate à 1% au sein de l'émulsion.

Le Triéthyl citrate est issu du processus d'estérification de l'acide citrique avec de l'éthanol. En cosmétique, cet ingrédient est fréquemment utilisé dans les déodorants comme alternatives aux sels d'aluminium. En raison de sa capacité à inhiber la décomposition enzymatique des composants de la sueur, il constitue un excellent ingrédient actif dans la lutte contre les odeurs corporelles.

EP1749919 divulgue une composition qui neutralise les odeurs et destinées à être appliquée sur les vêtements. Cette composition comprend du Zinc ricinoléate en combinaison avec du Triéthyl citrate.

EP1310234 divulgue des compositions déodorantes comprenant du diglycérol, du parfum, ainsi que des agents actifs antibactériens pouvant être du Zinc ricinoléate ou du Triéthyl citrate.

US2014/0322151 divulgue l'utilisation d'isorbide caprylates/caprates dans des compositions antitranspirantes et déodorantes. Ces compositions peuvent comprendre des substances actives contre les odeurs corporelles, lesdites substances pouvant être du Zinc ricinoléate ou du Triéthyl citrate.

US2006/0029624 et WO2006119981 divulguent des déodorants/antitranspirants sous forme de sticks basée sur une émulsion huile dans eau pouvant comprenant au moins un ingrédient actif déodorant ou antitranspirant. Le au moins un ingrédient actif peut être un inhibiteur d'enzymes tel que le Triéthyl citrate ou un absorbant d'odeurs tel que le Zinc ricinoléate.

La teneur en Zinc ricinoléate au sein des compositions déodorantes sous forme d'émulsions de l'art antérieur est généralement de 1% en masse.

EP3192491 ainsi que d'autres brevets au nom d'EVONIK divulguent des compositions cosmétiques déodorantes sous forme d'émulsion comprenant du Zinc ricinoléate à 1% en masse.

EP2179719 divulgue des compositions cosmétiques déodorantes sous forme d'émulsions qui peuvent contenir du Zinc ricinoléate comme ingrédient actif. Ce document divulgue que le Zinc ricinoléate est généralement présent au sein des compositions à des concentrations massiques comprises entre 0,1 et 1 %.

Une brochure commerciale de la société DR. STRAETMANS divulgue une composition déodorante sous forme de crème qui est une émulsion H/E comprenant 2% de zinc ricinoléate ainsi que 2% de triéthyl citrate. Cette émulsion est stable pendant plus de 3 mois à 20°C, 40°C et 4°C, cette formule comprend une quantité importante d'agents stabilisants (émulsionnants, alcool gras, gélifiants/épaississants). De par sa viscosité élevée de 25 000 cps, cette formule n'est pas susceptible d'être intégrée à un déodorant sous forme de bille ou roll-on.

EP1586304A1 divulgue une composition cosmétique déodorante de type émulsion E/H comprenant des émulsionnants qui sont des oligomères ou polymères dérivés de polyoléfine. La composition comprend un sel d'actif déodorant pouvant être un sel de zinc tel que le zinc ricinoléate. Les formules exemplifiées dans cette demande comprennent de l'aluminium chlorhydrate comme agent déodorant, composé qui est exclu de la présente invention.

US20090047226A1 divulgue une composition cosmétique pour roll-on sous forme d'une émulsion H/E comprenant des composés éthoxylés comme émulsionnants. La composition peut comprendre une substance active déodorante pouvant être un adsorbeur d'odeur tel que le zinc ricinoléate. La substance active déodorante peut être également un inhibiteur d'enzyme tel que le triéthyl citrate. Les formules exemplifiées dans cette demande comprennent de l'aluminium chlorhydrate comme agent déodorant, composé qui est exclu de la présente invention.

CN108619027A divulgue un shampoing déodorant comprenant du zinc ricinoléate ainsi que de l'octyl/décyl glycoside. Les formules divulguées ne sont pas des formules déodorantes pouvant être intégrées à un déodorant sous forme de roll-on.

FR3032613A1 divulgue une émulsion déodorante de type H/E comprenant comme émulsionnants des alkylpolyglucosides et un actif déodorant pouvant être un adsorbeur d'odeur tel que le zinc ricinoléate, il peut être également un inhibiteur d'enzyme tel que le triéthyl citrate. Les formules exemplifiées dans cette demande comprennent de l'aluminium chlorhydrate comme agent déodorant, composé qui est exclu de la présente invention.

De façon surprenante, la demanderesse a réussi à stabiliser une composition cosmétique déodorante sous forme d'une émulsion apte à être intégrée à un déodorant sous forme de bille ou roll-on comprenant une teneur en Zinc ricinoléate supérieure à 1% tout en utilisant une faible quantité d'agents stabilisants. Elle a réussi à atteindre une teneur en Zinc ricinoléate de l'ordre de 3% qui peut être normalement obtenue uniquement dans des compositions déodorantes sous forme solide anhydre ou dans des émulsions issues de recettes maison dont la stabilité dans le temps ne dépasse pas 3 mois.

La présente invention concerne une composition cosmétique déodorante sous forme d'émulsion stable apte à être intégrée dans un déodorant sous forme de bille ou roll-on comprenant du zinc ricinoléate à une teneur supérieure à 1% caractérisée en ce qu'elle ne contient pas de sels d'aluminium.
La présente invention concerne une composition cosmétique déodorante sous forme d'émulsion stable apte à être intégrée dans un déodorant sous forme de bille ou roll-on comprenant du zinc ricinoléate à une teneur supérieure à 1% caractérisée en ce qu'elle ne contient pas de chlorhydrate d'aluminium.

On entend par « apte à être intégrée dans un déodorant sous forme de bille ou roll-on », une émulsion qui possèdent des caractéristiques de viscosité lui permettant de pouvoir être intégrée à un déodorant sous forme de bille ou roll-on, ladite émulsion devant être ni trop liquide ni trop visqueuse. La viscosité optimale d'une telle émulsion doit être comprise entre 4000 et 8500 cps calculée à l'aide d'un viscosimètre Brookfield DVE Viscometer, mobile 63 à une vitesse de 10 tours.min-1. Cette plage est bien connue de l'Homme du métier.

On entend par « émulsion stable », une émulsion qui ne subit pas de changement d'aspect, de couleur, d'odeur, de pH, de viscosité et de taille et de forme de micelles à la fin de la période de mise en stabilité de 3 mois.

La mise en stabilité se fait en 2 étapes :
- Au lendemain de la fabrication de l'échantillon, l'émulsion subit un passage à la centrifugeuse pendant 15 min à 3500 tours.min-1. Cette étape équivaut à un vieillissement accéléré de 15 jours dans une étuve à 45°C. A la suite de cette étape, les émulsions stables ne présentant pas de déphasage et possédant des micelles de tailles et formes acceptables (réseau de micelles fin et homogène) sont aptes à être lancés en tests de stabilité suivants.
- Pendant 3 mois, des observations à J+7 ; J+17 ; J+14 ; J+21 ; J+28 ; J+2 mois et J+3 mois sont réalisées sur différents échantillons de l'émulsion qui permettent de juger de :
   ∘ La stabilité de l'émulsion à l'étuve à 45°C.
   ∘ La stabilité de l'émulsion au réfrigérateur à 6°C.
   ∘ La stabilité de l'émulsion aux UV.
   ∘ La stabilité de l'émulsion à température ambiante à l'abri de la lumière

Au cours de ces observations, le pH, la viscosité seront relevés et une observation des micelles au microscope sera réalisée.

Une fois les 3 mois de tests de stabilité validés, l'émulsion sera soumise à un challenge test microbiologique dont les résultats seront connus au bout de 28 jours.

Dans un mode de réalisation, la composition cosmétique déodorante selon l'invention est caractérisée en ce qu'elle ne subit pas de déphasage après un passage à la centrifugeuse pendant 15 min à 3500 tours.min-1.

Dans un mode de réalisation, la composition cosmétique déodorante selon l'invention est caractérisée en ce qu'elle est stable après un passage à l'étuve à 45°C pendant une durée minimum de 3 mois.

Dans un mode de réalisation, la composition cosmétique déodorante selon l'invention est caractérisée en ce qu'elle est stable après un passage au réfrigérateur à 6°C pendant une durée minimum de 3 mois.

Dans un mode de réalisation, la composition cosmétique déodorante selon l'invention est caractérisée en ce qu'elle est stable après avoir été exposée aux UV pendant une durée minimum de 3 mois.

Dans un mode de réalisation, la composition cosmétique déodorante selon l'invention est caractérisée en ce qu'elle est conservable pendant une durée supérieure à 3 mois.

Les noms des différents composés utilisés dans la présente invention sont donnés en noms INCI (International Nomenclature of Cosmetic Ingrédients) qui est une nomenclature obligatoire sur les produits cosmétiques depuis 1999. Créée en 1973 par une association américaine, la liste INCI a pour but de normer les ingrédients présents dans un produit cosmétique.

Dans un mode de réalisation, la composition cosmétique déodorante selon l'invention est caractérisée en ce qu'elle comprend de 1 à 5 % en masse de zinc ricinoléate par rapport à la masse totale de ladite composition.

Dans un mode de réalisation, la composition cosmétique déodorante selon l'invention est caractérisée en ce qu'elle comprend de 1 à 4 % en masse de zinc ricinoléate par rapport à la masse totale de ladite composition.

Dans un mode de réalisation, la composition cosmétique déodorante selon l'invention est caractérisée en ce que la teneur dudit zinc ricinoléate est supérieure à 2%.

Dans un mode de réalisation, la composition cosmétique déodorante selon l'invention est caractérisée en ce qu'elle comprend de 2 à 5 % en masse de zinc ricinoléate par rapport à la masse totale de ladite composition.

Dans un mode de réalisation, la composition cosmétique déodorante selon l'invention est caractérisée en ce qu'elle comprend de 2 à 4 % en masse de zinc ricinoléate par rapport à la masse totale de ladite composition.

Dans un mode de réalisation, la composition cosmétique déodorante selon l'invention est caractérisée en ce qu'elle comprend en outre au moins un émulsifiant huile dans eau.

On entend par « émulsifiant », un tensio-actif ou un groupe de tensio-actifs permettant de faciliter et stabiliser une émulsion. Au sein de la présente demande, les termes « émulsifiant » et « émulsionnant » sont synonymes et interchangeables.

Dans un mode de réalisation, la composition cosmétique déodorante selon l'invention est caractérisée en ce que ledit au moins un émulsifiant huile dans eau comprend au moins un tensio-actif non ionique.

Dans un mode de réalisation, la composition cosmétique déodorante selon l'invention est caractérisée en ce que ledit au moins un tensio-actif non ionique est choisi dans le groupe constitué des alkylpolyglucosides.

Les alkylpolyglucosides sont des tensio-actifs à base de sucres obtenus à partir d'un glucose et d'un alcool gras. Les émulsifiants à base d'alkylpolyglucosides peuvent être sous forme pure c'est à dire ne contenant que l'alkylpolyglucoside, ou sous forme mixte comprenant également l'alcool gras résiduel. Dans la présente invention, l'alkylpolyglucoside possède une chaine alkyl issue d'un alcool gras ainsi qu'une ou plusieurs unités glucose utilisables en cosmétique « BIO ».

Par exemple, la société SEPPIC commercialise des émulsifiants mixtes sous la marque MONTANOV™ utilisables dans la présente invention.

Dans un mode de réalisation, la composition cosmétique déodorante selon l'invention est caractérisée en ce que ledit au moins un tensio-actif non ionique est choisi dans le groupe constitué de l'Arachidyl Glucoside, du Myristyl Glucoside, du Coco-Glucoside, du Cetearyl Glucoside ou du C12-20 Alkyl Glucoside, seuls ou en combinaison.

Dans un mode de réalisation, la composition cosmétique déodorante selon l'invention est caractérisée en ce qu'elle comprend en outre de 0,5 à 6% en masse d'au moins un émulsifiant par rapport à la masse totale de ladite composition.

Dans un mode de réalisation, la composition cosmétique déodorante selon l'invention est caractérisée en ce qu'elle comprend en outre de 1 à 6% en masse d'au moins un émulsifiant par rapport à la masse totale de ladite composition.

Dans un mode de réalisation, la composition cosmétique déodorante selon l'invention est caractérisée en ce qu'elle comprend en outre de 2 à 4% en masse d'au moins un émulsifiant par rapport à la masse totale de ladite composition.

Dans un mode de réalisation, la composition cosmétique déodorante selon l'invention est caractérisée en ce qu'elle comprend en outre au moins un autre émulsifiant choisi dans le groupe constitué des alcools gras, des sels d'acides gras ou composés dérivés d'acides gras, des protéines végétales hydrolysées, lecithin, xanthan gum, glycerin, Stearamidopropyl Dimethylamine, seuls ou en combinaison.

Dans un mode de réalisation, la composition cosmétique déodorante selon l'invention est caractérisée en ce que lesdits sels d'acides gras ou composés dérivés d'acides gras sont choisis dans le groupe constitué du Glyceryl stearate, Glyceryl stearate SE, Sorbitan olivate, Glyceryl Stearate Citrate, Polyglyceryl-3 Stearate, Polyglyceryl-3 Cocoate, Polyglyceryl-4 Caprate, Polyglyceryl-4 Cocoate, Polyglyceryl-6 Caprylate, Polyglyceryl-6 Ricinoleate, Glyceryl Oleate, Glyceryl Oleate Citrate, Glyceryl Citrate, Lactate, Linoleate, Oleate, Sucrose Laurate, Sucrose Stearate, Sucrose laurate, Polyglyceryl-6 Stearate, Polyglyceryl-6 Behenate, polysorbate 80, Methyl glucose sesquistearate, Cetearyl olivate, seuls ou en combinaison.

Dans un mode de réalisation, la composition cosmétique déodorante selon l'invention est caractérisée en ce qu'elle comprend en outre de 0,05 à 5% en masse d'au moins un autre émulsifiant par rapport à la masse totale de ladite composition.

Dans un mode de réalisation, la composition cosmétique déodorante selon l'invention est caractérisée en ce qu'elle comprend en outre de 0,25 à 4% en masse d'au moins un autre émulsifiant par rapport à la masse totale de ladite composition.

Dans un mode de réalisation, la composition cosmétique déodorante selon l'invention est caractérisée en ce qu'elle comprend en outre de 0,5 à 2,5% en masse d'au moins un autre émulsifiant par rapport à la masse totale de ladite composition.

Dans un mode de réalisation, la composition cosmétique déodorante selon l'invention est caractérisée en ce qu'elle comprend en outre du Triéthyl citrate.

Dans un mode de réalisation, la composition cosmétique déodorante selon l'invention est caractérisée en ce qu'elle comprend en outre de 2 à 10 % en masse de Triéthyl citrate par rapport à la masse totale de ladite composition.

Dans un mode de réalisation, la composition cosmétique déodorante selon l'invention est caractérisée en ce qu'elle comprend en outre de 4 à 8 % en masse de Triéthyl citrate par rapport à la masse totale de ladite composition.

Dans un mode de réalisation, la composition cosmétique déodorante selon l'invention est caractérisée en ce qu'elle comprend en outre de 5 à 7 % en masse de Triéthyl citrate par rapport à la masse totale de ladite composition.

Dans un mode de réalisation, la composition cosmétique déodorante selon l'invention est caractérisée en ce qu'elle comprend en outre au moins un antibactérien.

On entend par « antibactérien », une substance ou un composé qui est actif contre les bactéries. Cette définition englobe les bactériostatiques qui agissent sur le développement des bactéries et les bactéricides qui tuent les bactéries.

Dans un mode de réalisation, la composition cosmétique déodorante selon l'invention est caractérisée en ce que le au moins un antibactérien est choisi dans le groupe constitué du Propanediol, Farnesol, Glyceryl caprylate, Salicylic acid, Zinc gluconate, Zinc PCA, Zinc citrate, Glycyrrhiza glabra root extract, Gluconolactone, Zinc oxide, Glyceryl caprate, Polyglyceryl-3 caprylate, Sodium usnate, Lactococcus ferment extract, Capryloyl glycine, Sodium silicate, Ethylhexylglycerin, Arginine, Arginine PCA, PCA ethyl cocoyl arginate, seuls ou en combinaison.

Dans un mode de réalisation, la composition cosmétique déodorante selon l'invention est caractérisée en ce que le au moins un antibactérien est choisi dans le groupe constitué du Glyceryl caprylate, Propanediol, Farnesol, Salicylic acid, Zinc citrate, Zinc PCA, Zinc gluconate, seuls ou en combinaison.

Dans un mode de réalisation, la composition cosmétique déodorante selon l'invention est caractérisée en ce que le au moins un antibactérien est le Zinc gluconate.

Dans un mode de réalisation, la composition cosmétique déodorante selon l'invention est caractérisée en ce qu'elle comprend en outre de 1 à 25 % en masse d'antibactérien par rapport à la masse totale de ladite composition.

Dans un mode de réalisation, la composition cosmétique déodorante selon l'invention est caractérisée en ce qu'elle comprend en outre de 5 à 20 % en masse d'antibactérien par rapport à la masse totale de ladite composition.

Dans un mode de réalisation, la composition cosmétique déodorante selon l'invention est caractérisée en ce qu'elle comprend en outre de 8 à 18 % en masse d'antibactérien par rapport à la masse totale de ladite composition.

Dans un mode de réalisation, la composition cosmétique déodorante selon l'invention est caractérisée en ce qu'elle comprend en outre au moins un autre antibactérien.

Dans un mode de réalisation, la composition cosmétique déodorante selon l'invention est caractérisée en ce qu'elle comprend en outre au moins deux autres antibactériens.

Dans un mode de réalisation, la composition cosmétique déodorante selon l'invention est caractérisée en ce qu'elle comprend en outre au moins un absorbant.

On entend par « absorbant », une substance ou un composé doté de capacité d'absorption de liquides.

Dans un mode de réalisation, la composition cosmétique déodorante selon l'invention est caractérisée en ce que le au moins un absorbant est choisi dans le groupe constitué du Silica, Diatomaceous earth, Bambusa arundinacea stem extract, Talc, Bentonite, Perlite, Kaolin, Iris germanica root, Charcoal powder, des amidons végétaux, seuls ou en combinaison.

Dans un mode de réalisation, la composition cosmétique déodorante selon l'invention est caractérisée en ce qu'elle comprend en outre de 0,1 à 7 % en masse d'absorbant par rapport à la masse totale de ladite composition.

Dans un mode de réalisation, la composition cosmétique déodorante selon l'invention est caractérisée en ce qu'elle comprend en outre de 0,5 à 5,5 % en masse d'absorbant par rapport à la masse totale de ladite composition.

Dans un mode de réalisation, la composition cosmétique déodorante selon l'invention est caractérisée en ce qu'elle comprend en outre de 1 à 4 % en masse d'absorbant par rapport à la masse totale de ladite composition.

Dans un mode de réalisation, la composition cosmétique déodorante selon l'invention est caractérisée en ce que le au moins un absorbant est de nature minérale.

Dans un mode de réalisation, la composition cosmétique déodorante selon l'invention est caractérisée en ce que le au moins un absorbant est un composé à base de silice.

Dans un mode de réalisation, la composition cosmétique déodorante selon l'invention est caractérisée en ce que le au moins un absorbant est un composé comprenant au moins 70% de silice.

Dans un mode de réalisation, la composition cosmétique déodorante selon l'invention est caractérisée en ce qu'elle comprend en outre au moins une huile végétale.

Dans un mode de réalisation, la composition cosmétique déodorante selon l'invention est caractérisée en ce que ladite au moins une huile végétale est extraite de toute partie de l'espèce végétale choisie dans le groupe constitué *de Cocos nucifera, Cyperus esculentus, Punica granatum, Prunus armeniaca, Crambe abyssinia, Euterpe oleracea, Prunus dulcis, Carapa guaianensis, Argania spinosa, Hippophae rhamnoides, Persea gratissima, Avena Sativa, Orbignya oleifera, Adansonia digitata, Mauritia flexuosa, Camelia sinensis, Camelina sativa, Carthamus tinctorius, Ribes nigrum, Prunus Cerasus, Cannabis sativa, Cucumis sativis, Gossypium herbaceum, Vaccinium macrocarpon, Balanites roxburghii, Opuntia ficus indica, Rubus idaeus, Passiflora incarnata, Triticum vulgare, Hibiscus sabdariffa, Plukenetia volubilis, Simmondsia chinensis, Pongamia glabra, Aleurites moluccana, Macadamia integrifolia, Sclerocarya birrea, Citrullus vulgaris, Corylus avellana, Bertholletia excelsa, Butyrospermum parkii, Olea europaea, Oenothera biennis, Elaeis guineensis, Vitis vinifera, Perilla frutescens, Caryocar coriaceum, Pentaclethra macroloba, Ricinus communis, Rosa rubiginosa, Pouteria sapota, Oryza sativa, Solanum lycopersicum, Calodendrum capense,* seuls ou en combinaison.

Dans un mode de réalisation, la composition cosmétique déodorante selon l'invention est caractérisée en ce qu'elle comprend en outre de 0,1 à 7% en masse d'huile végétale par rapport à la masse totale de ladite composition.

Dans un mode de réalisation, la composition cosmétique déodorante selon l'invention est caractérisée en ce qu'elle comprend en outre de 0,5 à 6% en masse d'huile végétale par rapport à la masse totale de ladite composition.

Dans un mode de réalisation, la composition cosmétique déodorante selon l'invention est caractérisée en ce qu'elle comprend en outre de 1 à 5% en masse d'huile végétale par rapport à la masse totale de ladite composition.

Dans un mode de réalisation, la composition cosmétique déodorante selon l'invention est caractérisée en ce qu'elle comprend en outre au moins un prébiotique.

On entend par « prébiotiques », des composés qui favorisent de façon sélective la croissance de certaines bactéries de type prébiotique.

Dans un mode de réalisation, la composition cosmétique déodorante selon l'invention est caractérisée en ce que le au moins un prébiotique est choisi dans le groupe constitué du Candida bombicola/glucose/methyl rapeseedate ferment, Saccharomyces ferment, Lactobacillus ferment, Arundinaria gigantea ferment filtrate, seuls ou en combinaison.

Dans un mode de réalisation, la composition cosmétique déodorante selon l'invention est caractérisée en ce qu'elle comprend en outre de 0,1 à 7 % en masse de prébiotique par rapport à la masse totale de ladite composition.

Dans un mode de réalisation, la composition cosmétique déodorante selon l'invention est caractérisée en ce qu'elle comprend en outre de 0,5 à 5 % en masse de prébiotique par rapport à la masse totale de ladite composition.

Dans un mode de réalisation, la composition cosmétique déodorante selon l'invention est caractérisée en ce qu'elle comprend en outre de 1 à 3 % en masse de prébiotique par rapport à la masse totale de ladite composition.

Les composés suivants sont exclus de la présente invention :
- Ethanol, Caprylyl glycol, la Poudre de pierre d'alun (Potassium alum) et les sels d'aluminium (Aluminium chlorhydrate) pour leurs effets controversés.
- Magnésium oxide qui déstabilise la formule.
- Les huiles essentielles bactéricides (palmarosa, tea tree, etc...) car la base déodorante doit être neutre d'odeurs.

Dans un mode de réalisation, la composition cosmétique déodorante selon l'invention est caractérisée en ce qu'elle ne contient pas de composé choisi dans le groupe constitué de Ethanol, Caprylyl glycol, Potassium alum, Aluminium chlorydrate, Magnesium oxide, seuls ou en combinaison.

Dans un mode de réalisation, la composition cosmétique déodorante selon l'invention est caractérisée en ce qu'elle ne contient pas dd'alool, l'aloccol étant de préférence l'éthanol dont le nom INCI est « alcohol ».

Dans un mode de réalisation, la composition cosmétique déodorante selon l'invention est caractérisée en ce qu'elle ne contient pas d'huiles essentielles bactéricides.

Dans un mode de réalisation, la composition cosmétique déodorante selon l'invention est caractérisée en ce qu'elle est neutre d'odeurs.

Tous les ingrédients utilisés pour la composition déodorante selon l'invention sont 100% d'origine naturelle. La composition est certifiée BIO Cosmos.

La présente invention concerne également un déodorant comprenant une composition cosmétique déodorante selon l'invention.

Dans un mode de réalisation, le déodorant selon la présente invention est caractérisé en ce qu'il est sous forme de bille ou roll-on.

Dans un mode de réalisation, le déodorant selon la présente invention est caractérisé en qu'il est sous forme de spray.

La présente invention concerne également un procédé de stabilisation d'une émulsion apte à être intégrée dans un déodorant sous forme de bille ou roll-on comprenant une teneur en Zinc ricinoléate supérieure à 1% caractérisé en ce qu'il comprend l'ajout d'au moins un émulsifiant huile dans eau à ladite émulsion.

Dans un mode de réalisation, le procédé de stabilisation d'une émulsion selon l'invention est caractérisé en ce que la teneur en Zinc ricinoléate est comprise entre 1 et 5 %.

Dans un mode de réalisation, le procédé de stabilisation d'une émulsion selon l'invention est caractérisé en ce que la teneur en Zinc ricinoléate est comprise entre 1 et 4 %.

Dans un mode de réalisation, le procédé de stabilisation d'une émulsion selon l'invention est caractérisé en ce que la teneur en Zinc ricinoléate est supérieure à 2%.

Dans un mode de réalisation, le procédé de stabilisation d'une émulsion selon l'invention est caractérisé en ce que la teneur en Zinc ricinoléate est comprise entre 2 et 5 %.

Dans un mode de réalisation, le procédé de stabilisation d'une émulsion selon l'invention est caractérisé en ce que la teneur en Zinc ricinoléate est comprise entre 2 et 4 %.

Dans un mode de réalisation, le procédé de stabilisation d'une émulsion selon l'invention est caractérisé en ce que ledit au moins un émulsifiant huile dans eau comprend au moins un tensio-actif non ionique.

Dans un mode de réalisation, le procédé de stabilisation d'une émulsion selon l'invention est caractérisé en ce que ledit au moins un tensio-actif non ionique est choisi dans le groupe constitué des alkylpolyglucosides.

Dans un mode de réalisation, le procédé de stabilisation d'une émulsion selon l'invention est caractérisé en ce que ledit au moins un tensio-actif non ionique est choisi dans le groupe constitué du Arachidyl Glucoside, Myristyl Glucoside, Coco-Glucoside, Cetearyl Glucoside, C12-20 Alkyl Glucoside, seuls ou en combinaison.

Dans un mode de réalisation, le procédé de stabilisation d'une émulsion selon l'invention est caractérisé en ce qu'il comprend l'ajout de 0,5 à 6% d'au moins un émulsifiant par rapport à la masse totale de ladite émulsion.

Dans un mode de réalisation, le procédé de stabilisation d'une émulsion selon l'invention est caractérisé en ce qu'il comprend l'ajout de 1 à 5% d'au moins un émulsifiant par rapport à la masse totale de ladite émulsion.

Dans un mode de réalisation, le procédé de stabilisation d'une émulsion selon l'invention est caractérisé en ce qu'il comprend l'ajout de 2 à 4% d'au moins un émulsifiant par rapport à la masse totale de ladite émulsion.

Dans un mode de réalisation, le procédé de stabilisation d'une émulsion selon l'invention est caractérisé en ce qu'il comprend en outre l'ajout d'au moins un autre émulsifiant choisi dans le groupe constitué des alcools gras, des sels d'acides gras ou composés dérivés d'acides gras, lecithin, xanthan gum, glycerin, Stearamidopropyl Dimethylamine, seuls ou en combinaison

Dans un mode de réalisation, le procédé de stabilisation d'une émulsion selon l'invention est caractérisé en ce que lesdits sels d'acides gras ou composés dérivés d'acides gras sont choisis dans le groupe constitué du Glyceryl stearate, Glycéryl stéarate SE, Sorbitan olivate, Glyceryl Stearate Citrate, Polyglyceryl-3 Stearate, Polyglyceryl-3 Cocoate, Polyglyceryl-4 Caprate, Polyglyceryl-4 Cocoate, Polyglyceryl-6 Caprylate, Polyglyceryl-6 Ricinoleate, Glyceryl Oleate, Glyceryl Oleate Citrate, Glyceryl Citrate, Lactate, Linoleate, Oleate, Sucrose Laurate, Sucrose Stearate, Sucrose laurate, Polyglyceryl-6 Stearate, Polyglyceryl-6 Behenate, polysorbate 80, Methyl glucose sesquistearate, Cetearyl olivate, seuls ou en combinaison.

Dans un mode de réalisation, le procédé de stabilisation d'une émulsion selon l'invention est caractérisé en ce qu'il comprend en outre l'ajout de 0,05 à 5% d'au moins un autre émulsifiant par rapport à la masse totale de ladite émulsion.

Dans un mode de réalisation, le procédé de stabilisation d'une émulsion selon l'invention est caractérisé en ce qu'il comprend en outre l'ajout de 0,25 à 4% d'au moins un autre émulsifiant par rapport à la masse totale de ladite émulsion.

Dans un Dans un mode de réalisation, le procédé de stabilisation d'une émulsion selon l'invention est caractérisé en ce qu'il comprend en outre l'ajout de 0,5 à 2,5% d'au moins un autre émulsifiant par rapport à la masse totale de ladite émulsion.

### Exemples

### Exemple 1 : Formulation d'une composition cosmétique (Formulation 1) déodorante selon l'invention

| **Phase** | **Composés nom INCI** | **% massique** |
|---|---|---|
| A | Aqua | 64,6750 |
| A | Aloe Barbadensis Leaf Juice Powder | 0,3250 |
| A | Aqua | 4,6000 |
| B | Xanthan qum | 0,3000 |
| B | Glycerin | 2,0000 |
| C | Arachidyl Alcohol, Behenyl Alcohol, Arachidyl Glucoside | 3,0000 |
| C | Cyperus Esculentus Root Oil | 2,0000 |
| C | Glyceryl Stearate SE | 0,6000 |
| C | Zinc Ricinoleate*, Aqua, Tocopherol, Glycine Soja Oil | 3,0000 |
| C | Triethyl Citrate | 5,9000 |
| C | Glyceryl caprylate | 0,3000 |
| C | Niqella sativa seed oil | 2,0000 |
| C | Sorbitan olivate | 0,5000 |
| D | Aqua, Glycerin, Sodium Levulinate, Sodium anisate | 4,0000 |
| D | Salix nigra bark extract | 3,7000 |
| D | Farnesol | 0,1000 |
| D | Levulinic Acid, Aqua, Glycerin, Sodium Levulinate | 0,3000 |
| D | Silica | 2,5000 |
| E | Citric acid | 0,2000 |

| | | |
|---|---|---|
| * Au minimum 98,5% | | |

Mode opératoire :
- Les composés de la phase A sont pesés et chauffés à T=80°C.
- Les composés de la phase B sont empâtés et ajoutés à la phase A à partir de T=60°C sous agitation forte à 1000 - 1500 tours.min-1, mobile rotor-stator, pendant 15 min.
- Séparément, les composés de la phase C sont pesés et chauffés à T=80°C.
- Quand les phases A+B et C sont à T=80°C, la phase C est mélangée sous forte agitation dans la phase A+B à 3000 tours.min-1, mobile rotor/stator, pendant 20 min.
- Quand T=50°C, l'émulsion est refroidie sous faible agitation à 400 tours.min-1, mobile hélice.
- Quand T=30°C, Les composés de la phase D sont ajoutés et mélangés 2 min entre chaque ajout à 400 tours.min-1, mobile hélice.
- Le pH est mesuré puis ajusté si besoin avec le composé E pour obtenir un pH compris entre 4,5-5,0.

### Contrôle organoleptique :

Aspect : émulsion semi-épaisse
Couleur : beige clair
Odeur : neutre
pH : 4,79 avec 0,4% d'acide citrique
Viscosité mesurée à J+1 à une température de T=20,6°C avec un mobile S63 tournant à 10 tours.min-1 = 6 132 cps

Test de stabilité à la centrifugeuse 15 min à 3500 tours.min-1: L'émulsion est stable à J+1 après fabrication, pas de déphasage observé, le réseau de micelles est fin et homogène.

### Exemple 2 : Formulation d'une composition cosmétique (Formulation 2) déodorante selon l'invention

| **Phase** | **Composés noms INCI** | **% massique** |
|---|---|---|
| A | Aqua | 64,6750 |
| A | Aloe Barbadensis Leaf Juice Powder | 0,3250 |
| A | Aqua | 4,6000 |
| B | Xanthan qum | 0,3000 |
| B | Glycerin | 2,0000 |
| C | Sorbitan olivate | 0,5000 |
| C | Triethyl Citrate | 5,9000 |
| C | Cyperus Esculentus Root Oil | 2,0000 |
| C | Glyceryl caprylate | 0,3000 |
| C | Zinc Ricinoleate*, Aqua, Tocopherol, Glycine Soja Oil | 3,0000 |
| C | Arachidyl Alcohol, Behenyl Alcohol, Arachidyl Glucoside | 3,0000 |
| C | Glyceryl Stearate SE | 0,6000 |
| D | Candida bombicola/g ucose/methyl rapeseedate ferment, Aqua, Potassium sorbate | 2,0000 |
| D | Levulinic Acid, Aqua, Glycerin, Sodium Levulinate | 0,3000 |
| D | Salix niqra bark extract | 3,7000 |
| D | Aqua, Glycerin, Sodium Levulinate, Sodium anisate | 4,0000 |
| D | Farnesol | 0,1000 |
| D | Silica | 2,5000 |
| E | Citric acid | 0,2000 |

| | | |
|---|---|---|
| * Au minimum 98,5% | | |

### Mode opératoire :

- Les composés de la phase A sont pesés et chauffés à T=80°C.
- Les composés de la phase B sont empâtés et ajoutés à la phase A à partir de T=60°C sous agitation forte à 1000 - 1500 tours.min-1, mobile rotor-stator,pendant 15 min.
- Séparément, les composés de la phase C sont pesés et chauffés à T=80°C.
- Quand les phases A+B et C sont à T=80°C, la phase C est mélangée sous forte agitation dans la phase A+B à 3000 tours.min-1, mobile rotor stator,pendant 20 min.
- Quand T=50°C, l'émulsion est refroidie sous faible agitation à 400 tours.min-1, mobile hélice.
- Quand T=30°C, Les composés de la phase D sont ajoutés et mélangés 2 min entre chaque ajout à 400 tours.min-1, mobile hélice.
- Le pH est mesuré puis ajusté si besoin avec le composé E pour obtenir un pH compris entre 4,5-5,0.

### Contrôle organoleptique :

Aspect : émulsion semi-fluide
Couleur : beige clair
Odeur : neutre
pH : 5,46 sans ajustement, 4,92 avec 0,2% d'acide citrique
Viscosité mesurée à J+3 à une température de 22,8°C avec un mobile S63 tournant à 10 tours.min-1: 4 056 cps
Viscosité mesurée à J+4 à une température de 21,8°C avec un mobile S63 tournant à 10 tours.min-1: 3 996 cps

Test de stabilité à la centrifugeuse 15 min à 3500 tours.min-1: L'émulsion est stable à J+3 et J+4 après fabrication, pas de déphasage observé, le réseau de micelles est fin et homogène.

### Exemple 3 : Formulation d'une composition cosmétique (Formulation 3) déodorante selon l'invention

| **Phase** | **Composés noms INCI** | **% massique** |
|---|---|---|
| A | Phytic Acid, Aqua | 0,1000 |
| A | Aqua | 3,2000 |
| A | Aqua | 54,7250 |
| A | Aloe Barbadensis Leaf Juice Powder | 0,2750 |
| A | Propanediol | 10,0000 |
| B | Xanthan qum | 0,3000 |
| C | Glyceryl Stearate SE | 1,2000 |
| C | Glyceryl caprylate | 1,0000 |
| C | Cocos Nucifera Oil | 2,0000 |
| C | Cyperus Esculentus Root Oil | 2,0000 |
| C | Triethyl Citrate | 5,9000 |
| C | Arachidyl Alcohol, Behenyl Alcohol, Arachidyl Glucoside | 3,0000 |
| C | Zinc Ricinoleate*, Aqua, Tocopherol, Glycine Soja Oil | 3,0000 |
| D | Salix niqra bark extract | 3,0000 |
| D | Aqua, Glycerin, Sodium Levulinate, Sodium anisate | 4,0000 |
| D | Levulinic Acid, Aqua, Glycerin, Sodium Levulinate | 0,3000 |
| D | Zinc qluconate | 3,0000 |
| D | Diatomaceous Earth | 2,0000 |
| E | Lactic acid, Aqua | 1,0000 |

| | | |
|---|---|---|
| * Au minimum 98,5% | | |

### Mode opératoire :

- Les composés de la phase A sont pesés et chauffés à T=80°C.
- Le composé de la phase B est ajouté à la phase A à partir de T=60°C sous agitation forte, mobile rotor stator à 1000 - 1500 tours.min-1 pendant 15 min.
- Séparément, les composés de la phase C sont pesés et chauffés à T=80°C.
- Quand les phases A+B et C sont à T=80°C, C est mélangée sous forte agitation dans A+B, mobile rotor stator à 3000 tours.min-1 pendant 20 min.
- Quand T=50°C, l'émulsion est refroidie sous faible agitation, à l'aide d'un mobile hélice à 400 tours.min-1.
- Quand T=30°C, les composés de la phase D sont ajoutés et mélangés 2 min entre chaque ajout, à l'aide d'un mobile hélice à 400 tours.min-1.
- Dès la fin de l'introduction des composés de la phase D, le mélange est agité sous forte agitation, mobile rotor-stator à 1500-2000 tours.min-1 pendant 10 min afin de bien disperser D.
- Le pH est mesuré et ajusté si besoin avec la phase E pour obtenir un pH compris entre 4,8-5,3.

### Contrôle organoleptique et physico chimique :

Aspect : émulsion semi-fluide
Couleur : blanc cassé
Odeur : neutre
pH : 5,02 avec 0,4% acide lactique
Viscosité mesurée à J+1 à une température de 21°C avec un mobile S63 tournant à 30 tours.min-1 : 2088 cps
Viscosité mesurée à J+1 à une température de 21,2°C avec un mobile S63 tournant à 10 tours.min-1 : 7980 cps

Test de stabilité à la centrifugeuse 15 min à 3500 tours.min-1 : L'émulsion est stable, pas de déphasage observé, le réseau de micelles est fin et homogène.

### Exemple 4 : Formulation d'une composition cosmétique (Formulation 4) déodorante selon l'invention

| **Phase** | **Composés noms INCI** | **% massique** |
|---|---|---|
| A | Zinc Citrate | 1,0000 |
| A | Aqua | 64,6750 |
| A | Aqua | 3,6000 |
| A | Aloe Barbadensis Leaf Juice Powder | 0,3250 |
| B | Xanthan qum | 0,3000 |
| B | Glycerin | 2,0000 |
| C | Zinc Ricinoleate*, Aqua, Tocopherol, Glycine Soia Oil | 3,0000 |
| C | Triethyl Citrate | 5,9000 |
| C | Niqella sativa seed oil | 2,0000 |
| C | Glyceryl Stearate SE | 0,6000 |
| C | Cyperus Esculentus Root Oil | 2,0000 |
| C | Glyceryl caprylate | 0,3000 |
| C | Arachidyl Alcohol, Behenyl Alcohol, Arachidyl Glucoside | 3,0000 |
| C | Sorbitan olivate | 0,5000 |
| D | Salix niqra bark extract | 3,7000 |
| D | Farnesol | 0,1000 |
| D | Aqua, Glycerin, Sodium Levulinate, Sodium anisate | 4,0000 |
| D | Levulinic Acid, Aqua, Glycerin, Sodium Levulinate | 0,3000 |
| D | Silica | 2,5000 |
| E | Citric acid | 0,2000 |

| | | |
|---|---|---|
| * Au minimum 98,5% | | |

### Mode opératoire :

- Les composés de la phase A sont pesés et chauffés à T=80°C.
- Les composés de la phase B sont empâtés et sont ajoutés à la phase A à partir de T=60°C sous agitation forte, mobile rotor stator à 1000 - 1500 tours.min⁻¹ pendant 15 min.
- Séparément, les composés de la phase C sont pesés et chauffés à T=80°C.
- Quand les phases A+B et C sont à T=80°C, C est mélangée sous forte agitation dans A+B, mobile rotor stator à 3000 tours.min-1 pendant 20 min.
- Quand T=50°C, l'émulsion est refroidie sous faible agitation, à l'aide d'un mobile hélice à 400 tours.min-1.
- Quand T=30°C, les composés de la phase D sont ajoutés et mélangés 2 min entre chaque ajout, à l'aide d'un mobile hélice à 400 tours.min-1.
- Le pH est mesuré et ajusté si besoin avec le composé de la phase E pour obtenir un pH compris entre 4,5-5,0.

### Contrôle organoleptique et physico chimique :

Aspect : émulsion semi-fluide
Couleur : beige clair
Odeur : neutre
pH : 5,61 sans ajustement, 4,70 avec 0,3% d'acide citrique
Viscosité mesurée à J+2 à une température de 21,6°C avec un mobile S63 tournant à 10 tours.min-1: 4200 cps

Test de stabilité à la centrifugeuse 15 min à 3500 tours.min-1: L'émulsion est stable, les clichés microscopiques des micelles sont corrects, pas d'observations de la dégradation de la taille et de l'homogénéité des micelles après centrifugation.

### Exemple 5 : Formulation d'une composition cosmétique (Formulation 5) déodorante selon l'invention

| **Phase** | **Composés noms INCI** | **% massique** |
|---|---|---|
| A | Aloe Barbadensis Leaf Juice Powder | 0,3250 |
| A | Aqua | 64,6750 |
| B | Xanthan qum | 0,3000 |
| B | Glycerin | 2,0000 |
| C | Glyceryl caprylate | 0,3000 |
| C | Sorbitan Olivate | 0,5000 |
| C | Arachidyl Alcohol, Behenyl Alcohol, Arachidyl Glucoside | 3,0000 |
| C | Cyperus Esculentus Root Oil | 2,0000 |
| C | Glyceryl Stearate SE | 0,6000 |
| C | Triethyl Citrate | 5,9000 |
| C | Zinc Ricinoleate*, Aqua, Tocopherol, Glycine Soja Oil | 3,0000 |
| D | Levulinic Acid, Aqua, Glycerin, Sodium Levulinate | 0,3000 |
| D | Aqua, Glycerin, Sodium Levulinate, Sodium anisate | 4,0000 |
| D | Farnesol | 0,1000 |
| D | Salix niqra bark extract | 3,7000 |
| D | Silica | 2,5000 |
| E | Zinc PCA | 1,0000 |
| E | Aqua | 5,6000 |
| F | Citric acid | 0,2000 |

| | | |
|---|---|---|
| * Au minimum 98,5% | | |

### Mode opératoire :

- Les composés de la phase A sont pesés et chauffés à T=80°C.
- Les composés de la phase B sont empâtés et sont ajoutés à la phase A à partir de T=60°C sous agitation forte, mobile rotor stator à 1000 - 1500 tours.min-1 pendant 15 min.
- Séparément, les composés de la phase C sont pesés et chauffés à T=80°C.
- Quand les phases A+B et C sont à T=80°C, C est mélangée sous forte agitation dans A+B, mobile rotor stator à 3000 tours.min-1 pendant 20 min.
- Quand T=50°C, l'émulsion est refroidie sous faible agitation, à l'aide d'un mobile hélice à 400 tours.min-1.
- Quand T=30°C, les composés de la phase D sont ajoutés et mélangés 2 min entre chaque ajout, à l'aide d'un mobile hélice à 400 tours.min-1.
- Les composés de la phase E sont solubilisés et ajoutés à A+B+C+D, l'ensemble est mélangé 2 min entre chaque ajout, à l'aide d'un mobile hélice à 400 tours.min-1.
- Le pH est mesuré et ajusté si besoin avec le composé de la phase F pour obtenir un pH compris entre 4,5-5,0.

### Contrôle organoleptique et physico chimique :

Aspect : émulsion semi-fluide
Couleur : beige clair
Odeur : neutre
pH : 5,38 sans ajustement, 4,75 avec 0,2% d'acide citrique
Viscosité mesurée à J+1 à une température de 21,6°C avec un mobile S63 tournant à 10 tours.min-1: 5976 cps

Test de stabilité à la centrifugeuse 15 min à 3500 tours.min-1: L'émulsion est stable, les clichés microscopiques des micelles sont corrects, pas d'observations de la dégradation de la taille et de l'homogénéité des micelles après centrifugation.

### Exemple 6 : Tests d'usaae des formulations 3, 4 et 5 selon l'invention afin de déterminer l'efficacité de 3 différents antibactériens sous forme de sels de zinc (autres que le zinc ricinoleate qui est l'actif principal).

La formulation 3 contient du zinc gluconate, la formulation 4 du zinc citrate et la formulation 5 du zinc PCA. Le Zinc ricinoléate et le triéthyl citrate sont en proportions égales dans ces 3 formules.

Des tests d'usage ont été réalisés pour les formulations 3, 4 et 5 afin de déterminer l'appréciation de l'efficacité de ces formules en utilisation réelle.

Pour chaque formule le panel était constitué de 10 volontaires, 7 femmes et 3 hommes, âgés entre 20 et 55 ans, et de tout type de peau. Les femmes enceintes ou allaitantes sont exclues des critères d'inclusion des études.

Chaque formule testée est appliquée sous les aisselles une fois par jour pendant une semaine.

Pour la formulation 3, 90% des panélistes jugent le produit efficace et 90% se disent prêts à acheter le produit.

Pour la formulation 4, 30% des panélistes jugent le produit efficace et 10% se disent prêts à acheter le produit.

Pour la formulation 5, 20% des panélistes jugent le produit efficace et 10% se disent prêts à acheter le produit.

Ces tests ont donc permis de mettre en évidence que le zinc gluconate comme actif antibactérien est plus performant et efficace que le zinc PCA ou le zinc citrate.

### Contre-exemple 1 : recette « maison » AROMA ZONE déodorant crème roll-on zinc et bergamote

| **Phase** | **Ingrédients** | **% massique estimé** |
|---|---|---|
| A | Agent émollient Caprylis | 25,00 |
| A | Actif cosmétique Zinc ricinoleate | 3,00 |
| B | Emulsifiant Gélisucre BIO | 10,00 |
| C | Gomme Xanthane | 0,5 |
| C | Microsphères de silice | 4,00 |
| C | Eau minérale | 45,50 |
| D | Huile essentielle bergamote sans furocoumarines BIO | 2,00 |
| D | Complexe déodorant Farnesol - Lemonester | 6,00 |
| D | Conservateur Leucidal | 4,00 |

### Mode opératoire préconisé :

- Faire fondre la phase A dans un bol au bain-marie (70-80°C), puis placer le bol dans un bain d'eau froide jusqu'à refroidissement.
- Dans un deuxième bol, mettre la phase B.
- Dans un troisième bol, mettre la phase C
- Ajouter petit à petit la phase A à la phase B et mélanger énergiquement entre chaque ajout au mini-fouet. Le mélange se gélifie.
- Mélanger la phase C, puis l'incorporer au mélange des phases A + B.
- Ajouter la phase D.
- Transvaser la préparation dans le roll-on.

Le pH de cette préparation est de 6,0-7,0.

La préconisation de conservation du déodorant crème roll-on zinc et bergamote est de 3 mois environ à l'abri de la lumière et de la chaleur.

Le test de stabilité à 1 mois a montré pour l'échantillon en étuve à T=45°C, un déphasage ainsi qu'une texture plus liquide.

### Contre-exemple 2 : Formulation d'une composition cosmétique (Formulation 4) déodorante

| **Phase** | **Composés noms INCI** | **% massique** |
|---|---|---|
| A | Aloe Barbadensis Leaf Juice Powder | 0,3250 |
| A | Aqua | 4,1000 |
| A | Aqua | 64,6750 |
| A | Phytic Acid, Aqua | 0,1000 |
| A | Glycerin | 2,0000 |
| B | Hydrolyzed Vegetable Protein, Sodium Citrate, Magnesium Stearate, Xanthan Gum | 3,0000 |
| C | Cyperus Esculentus Root Oil | 2,0000 |
| C | Glyceryl Stearate SE | 0,5000 |
| C | Glyceryl caprylate | 0,3000 |
| C | Cocos Nucifera Oil | 2,0000 |
| C | Zinc Ricinoleate, Aqua, Tocopherol, Glycine Soia Oil | 3,0000 |
| C | Triethyl Citrate | 5,9000 |
| D | Aqua, Glycerin, Sodium Levulinate, Sodium anisate | 4,0000 |
| D | Farnesol | 0,1000 |
| D | Levulinic Acid, Aqua, Glycerin, Sodium Levulinate | 0,3000 |
| D | Salix niqra bark extract | 3,7000 |
| D | Zinc qluconate | 2,0000 |
| D | Diatomaceous Earth | 2,0000 |

### Mode opératoire :

Les composés de la phase A sont pesés et chauffés à T=80°C.

A T=80°C, le composé de la phase B est ajouté sous forte agitation à 1000 tours.min-1 pendant 10 min.

Séparément, les composés de la phase C sont pesés et chauffés à T=80°C.

Quand les phases A+B et C sont à T=80°C, C est mélangée sous forte agitation dans A+B à 3000 tours.min-1 pendant 20 min.

Quand T=50°C, l'émulsion est refroidie sous faible agitation à 400 tours.min-1.

Quand T=30°C, Les composés de la phase D sont ajoutés et mélangés 2 min entre chaque ajout à 400 tours.min-1.

Dès la fin de l'introduction des composés de la phase D, le mélange est agité sous forte agitation, mobile rotor-stator à 1500-2000 tours.min-1 pendant 10 min afin de bien disperser D.

Le pH est mesuré et ajusté au besoin avec de l'acide lactique pour obtenir un pH compris entre 4,5-5,0.

### Contrôle organoleptique et physico chimique :

Aspect : émulsion épaisse et très gélifiée
Couleur : jaune clair / beige foncé
Odeur : végétale
pH : 5,92 avant ajustement, 4,46 avec 1,8% d'acide lactique

Test de stabilité à la centrifugeuse 15 min à 3500 tours.min-1 : L'émulsion semble stable à J+1, un léger dépôt de poudre est observé au fond du tube à essai. L'observation des micelles au microscope révèle que celle-ci sont grossières et espacées, le réseau de micelles devant être homogène et fin pour une stabilité correcte. La présence du Zinc ricinoléate à 3% déstabilise la formule.

## Revendications

1. Composition cosmétique déodorante sous forme d'émulsion stable apte à être intégrée dans un déodorant sous forme de bille ou roll-on comprenant du zinc ricinoleate à une teneur supérieure à 1% **caractérisée en ce qu'**elle ne contient pas de sels d'aluminium..

2. Composition cosmétique déodorante selon la revendication 1, **caractérisée en ce qu'**elle ne contient pas de chlorhydrate d'aluminium

3. Composition cosmétique déodorante l'une quelconque des revendications précédentes,**caractérisée en ce qu'**elle comprend en outre au moins un émulsifiant huile dans eau.

4. Composition cosmétique déodorante selon la revendication 3, **caractérisée en ce que** ledit au moins un émulsifiant huile dans eau comprend au moins un tensio-actif non ionique.

5. Composition cosmétique déodorante selon la revendication 4, **caractérisée en ce que** ledit au moins un tensio-actif non ionique est choisi dans le groupe constitué des alkylpolyglucosides.

6. Composition cosmétique déodorante selon l'une quelconque des revendications 3 à 5, **caractérisée en ce qu'**elle comprend en outre au moins un autre émulsifiant choisi dans le groupe constitué des alcools gras, des sels d'acides gras ou composés dérivés d'acides gras, des protéines végétales hydrolysées, lecithin, xanthan gum, glycerin, Stearamidopropyl Dimethylamine, seuls ou en combinaison.

7. Composition cosmétique déodorante selon l'une quelconque des revendications précédentes, **caractérisée en ce qu'**elle comprend en outre du triéthyl citrate.

8. Composition cosmétique déodorante selon l'une quelconque des revendications précédentes, **caractérisée en ce qu'**elle comprend en outre de 2 à 10 % en masse de Triethyl citrate par rapport à la masse totale de ladite composition.

9. Composition cosmétique déodorante selon l'une quelconque des revendications précédentes, **caractérisée en ce qu'**elle comprend en outre au moins un antibactérien.

10. Composition cosmétique déodorante selon la revendication 9, **caractérisée en ce que** le au moins un antibactérien est le Zinc gluconate.

11. Composition cosmétique déodorante selon l'une quelconque des revendications précédentes **caractérisée en ce qu'**elle comprend en outre au moins un absorbant.

12. Déodorant comprenant une composition cosmétique déodorante selon l'une quelconque des revendications précédentes.

13. Déodorant selon la revendication 12 **caractérisé en ce qu'**il est sous forme de bille ou roll-on.

14. Procédé de stabilisation d'une émulsion apte à être intégrée dans un déodorant sous forme de bille ou roll-on comprenant une teneur en Zinc ricinoleate supérieure à 1% **caractérisé en ce qu'**il comprend l'ajout d'au moins un émulsifiant huile dans eau à ladite émulsion.

15. Procédé de stabilisation d'une émulsion selon la revendication 14, **caractérisé en ce que** ledit au moins un émulsifiant huile dans eau comprend au moins un tensio-actif non ionique.

16. Procédé de stabilisation d'une émulsion selon la revendication 15, **caractérisé en ce que** ledit au moins un tensio-actif non ionique est choisi dans le groupe constitué des alkylpolyglucosides.
